# EUROPEAN PATENT APPLICATION

(11) **EP 3 751 576 A1**
(43) Date of publication of application: **16.12.2020**
(21) Application number: 19199919.2
(22) Date of filing: 26.09.2019
(51) Int. Cl.: G16H 20/13, A61M 35/00, B01F 13/00, B01F 15/02

(54) **INTELLIGENT SKIN BEAUTY SYSTEM AND USER TERMINAL**

(30) Priority: 10.06.2019 CN 201910497873
(71) Applicant: Tronxyz Intelligence Co., Ltd., Mountain View, California 94043 (US)
(72) Inventor: Guo, Fuzhong, Shenzhen 518000 (CN); Song, Lei, Shenzhen 518000 (CN); Quan, Hongwei, Shenzhen 518000 (CN)
(74) Representative: Lohr, Jöstingmeier & Partner

(57) **Abstract**

A beauty care system is provided. The beauty care system includes a communication module for establishing a communication link with a user terminal; and a beauty-care-solution-mixing control module for receiving control information from the user terminal over the communication link and performing a beauty-care operation on a target object based on the control information from the user terminal. The control information is determined based on at least one of information of an electronic tag of a cosmetic capsule and characteristic information of the target object.

## Description

### FIELD OF TECHNOLOGY

The present disclosure generally relates to the field of beauty systems and, more particularly, relates to an intelligent skin beauty system and a user terminal.

### BACKGROUND

As people's living standards continue to improve, people's requirements for facial care and beauty care are also increasing. The beauty care system is a device that relies on physical intervention to soothe the skin, better cleanse and absorb skin care products.

Commonly used beauty care systems are usually stand-alone beauty equipment, or a desktop device with multiple operating heads on one main unit, or multiple functions integrated on one main unit. Currently, the user is often standardized for these beauty care systems, and the beauty care systems cannot take differentiated and targeted beauty care measures for different users.

The disclosed systems and methods are directed to solving at least partial problems set forth above and other problems.

### BRIEF SUMMARY OF THE DISCLOSURE

One aspect of the present disclosure provides a beauty care system. The beauty care system may include a communication module for establishing a communication link with a user terminal. Preferably it as well includes a beauty-care-solution-mixing control module for receiving control information from the user terminal over the communication link and optionally performing a beauty-care operation on a target object based on the control information from the user terminal. The control information may be determined based on at least one of information of an electronic tag of a cosmetic capsule and characteristic information of the target object.

Another aspect of the present disclosure provides a beauty care method for a user terminal. The beauty care method may include establishing a communication link with a beauty care system, and it may include determining information of an electronic tag of a cosmetic capsule. The information may include at least one of information on whether the cosmetic capsule can be used and/or what kind of beauty care mode is recommended for using the beauty capsule. The beauty care method may also include obtaining characteristic information of a target object; and preferably based on at least one of the information of the electronic tag and the characteristic information of the target object, determining control information for the beauty care system to perform a beauty-care operation on the target object. Optionally the control information may be transmitted to the beauty care system. Optionally transmitting the control information may cause the beauty care system to perform the beauty-care operation on the target object.

Other aspects of the present disclosure can be understood by those skilled in the art in light of the description, the claims, and the drawings of the present disclosure.

### BRIEF DESCRIPTION OF THE DRAWINGS

In order to more clearly illustrate technical solutions in disclosed embodiments of the present invention, drawings necessary for the description of the embodiments or the prior art are briefly introduced below. Obviously, the drawings described below are only some embodiments of the present disclosure, and it is possible for those ordinarily skilled in the art to derive other drawings from these drawings without creative effort.
FIG. 1 illustrates an operation environment incorporating certain embodiments of the present disclosure;
FIG. 2 illustrates an exemplary computing system for implementing a terminal consistent with embodiments of the present disclosure;
FIG. 3 illustrates certain functional modules in a beauty care system consistent with embodiments of the present disclosure;
FIGS. 4-6 illustrate an exemplary beauty system incorporating various functional modules consistent with embodiments of the present disclosure;
FIG. 7A illustrates an exemplary beauty care method for a terminal consistent with certain embodiments of the present disclosure;
FIG. 7B illustrates a process of determining the control information of a beauty care operation of a target object consistent with the embodiments of the present disclosure;
FIG. 7C illustrates a skin beauty care method for a beauty system consistent with the embodiments of the present disclosure;
FIG. 8 illustrates another skin beauty care method for a beauty system consistent with the embodiments of the present disclosure;
FIGS. 9-12 illustrate certain skin beauty care methods for a terminal and a beauty system consistent with embodiments of the present disclosure;
FIG. 13 illustrates an exemplary operation process based on a beauty capsule integrated with an electronic tag consistent with the embodiments of the present disclosure;
FIG. 14 illustrates an exemplary block diagram of a terminal consistent with the embodiments of the present disclosure; and
FIG. 15 illustrates an exemplary block diagram of a beauty system consistent with the embodiments of the present disclosure.

### DETAILED DESCRIPTION

The technical solutions in the embodiments of the present disclosure will be described as follows with reference to the accompanying drawings. Obviously, the embodiments described below are merely a part of, rather than entire, embodiments of the present invention. On the basis of the disclosed embodiments, other embodiments obtainable by those ordinarily skilled in the art without creative effort shall fall within the scope of the present invention.

In the embodiments of the present invention, the words "exemplary" or "such as" are used to mean an example, illustration, or illustration. Any embodiment or design described as "exemplary" or "for example" in the embodiments of the invention should not be construed as preferred or advantageous over other embodiments or designs. Rather, the use of the words "exemplary" or "such as" is intended to present the concepts in a particular manner.

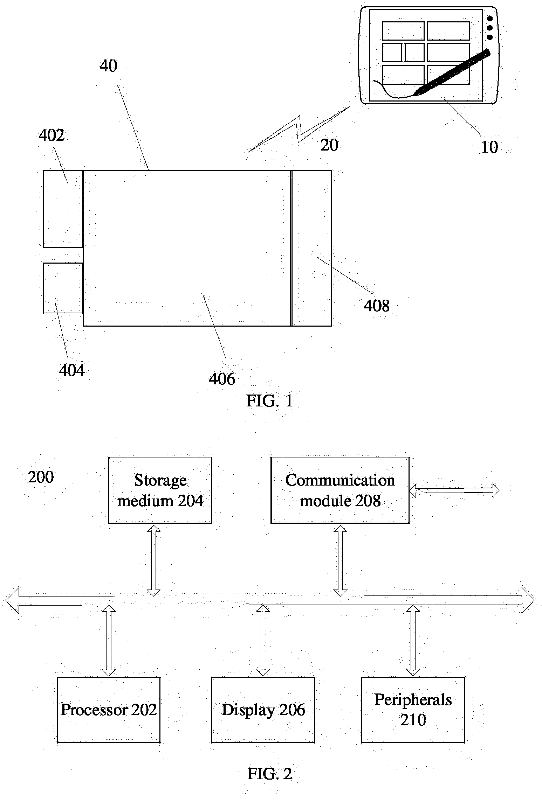

FIG. 1 illustrates an operation environment incorporating certain embodiments of the present disclosure. As shown in FIG. 1, the operation environment may include a user terminal 10 (or simply terminal 10) and optionally an intelligent skin beauty system 40 (or simply as beauty system 40). The terminal 10 and the beauty system 40 may be connected through a link 20, which may be a wireless communication link, such as Cellular, Bluetooth, Wireless Fidelity (WiFi), or Near Field Communication (NFC) connections, etc. Link 20 may also be a wired connection or a computer network connection, such as the Internet. Although only one terminal and one beauty system are shown in FIG. 1, any number of these components may be included.

The terminal 10 may include any appropriate type of user computing device, such as a mobile phone, a tablet computer, a notebook computer, an Ultra-Mobile Personal Computer (UMPC), a netbook or a personal digital assistant (PDA), and a mobile internet device (Mobile). Internet Device, MID), wearable device, or in-vehicle device, etc. FIG. 2 illustrates an exemplary computing system for implementing the terminal 10.

As shown in FIG. 2, computing system 200 may include at least of a processor 202, a storage medium 204, a display 206, a communication module 208, and peripherals 210. Certain devices may be omitted and other devices may be included.

Processor 202 may include any appropriate processor or processors, such as a general-purpose central computing unit (CPU), a graphic processing unit (GPU), or an application-specific integrated circuit (ASIC), a microcontroller, etc. Further, processor 202 can include multiple cores for multi-thread or parallel processing. Storage medium 204 may include memory modules, such as ROM, RAM, flash memory modules, and mass storages, such as CD-ROM and hard disk, etc. Storage medium 204 may store computer programs for implementing various processes, when the computer programs may be executed by processor 202.

Further, peripherals 210 may include various sensors and other I/O devices, such as cameras, a touch screen, keyboard, or mouse, etc., and communication module 208 may include certain network interface devices for establishing connections through communication networks.

The beauty system 40 may include various types skin beauty systems, including a radio frequency beauty system, a photon beauty system, a micro-current beauty system, etc. The radio frequency beauty system stimulates the collagen proliferation under the dermis layer of the skin, thereby achieving the effect of skin rejuvenation, reducing wrinkles, and lifting the facial skin. The photon beauty system uses strong pulse light to directly irradiate the skin to produce photo-biochemical effects. On the one hand, collagen fibers and elastic fibers in the skin can be re-arranged to restore skin elasticity and improve facial microcirculation, eliminating or reducing wrinkles. On the other hand, the strong pulse light can penetrate the skin, and is preferentially absorbed by the pigment clusters and capillaries in the tissue. The heat can coagulate the blood without destroying the normal skin tissue, and the pigment group is destroyed by thermal decomposition, thereby treating the capillary expanding and remove stains. The micro-current beauty system can speed up the introduction of skin care products such as beauty care solutions, thus helping the skin to better absorb the essence of skin care products. Other types of beauty systems may also be used.

As shown in FIG. 1, the beauty system 40 may include at least one of one or more sensor 402, one or more actuator 404, a body 406, and a power supply 408. Other components may also be included.

The sensor 402 may include any appropriate type of sensor for detecting one or more parameters to be used by the beauty system 40. For example, the sensor 402 may be an optical detector for detecting skin characteristics based on light. The actuator 404 may be any device that can be activated to perform a certain function, such as skin care action or massage movement, etc. Further, body 406 may house various control units and/or functional modules of the beauty system 40, as well as the sensor 402 and the actuator 404. For example, body 406 may include a microcontroller coupled with memory for controlling system operation of the beauty system 40. The power supply 408 may include any direct current (DC) source, such as a battery, or any alternate current (AC) source.

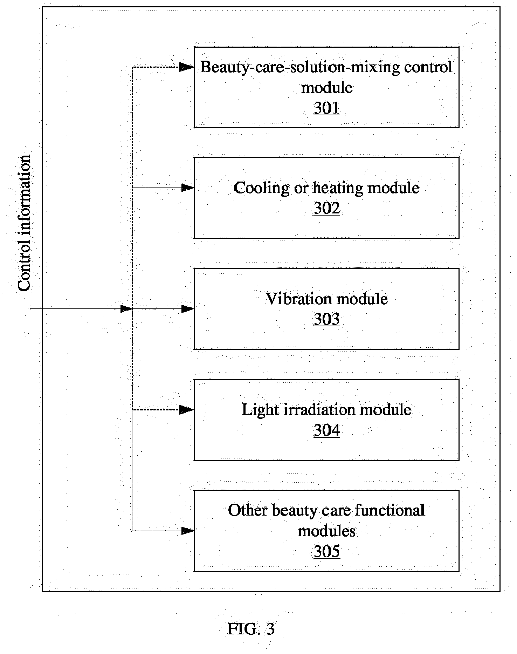

FIG. 3 illustrates certain functional modules in the beauty system 40 consistent with the disclosed embodiments of the present disclosure. As shown in FIG. 3, the beauty system 40 may include at least one of a beauty-care-solution-mixing control module 301, a cooling or heating module 302, a vibration module 303, a light emitting module 304, and other beauty care functional modules 305. It can be understood that any number and types of functional modules may be included in the beauty system.

In certain embodiments, the beauty-care-solution-mixing control module 301 may be configured to perform a beauty-care operation of the beauty-care-solution mixture based on control information. For example, the control information may include the information from an electronic tag of a cosmetic capsule obtained by a user of the beauty system 40 or the terminal 10. The control information may also include characteristic information of a target object, such as a user (e.g., a skin region of the user). The control information may also include any further control information determined based on the information from the electronic tag of the cosmetic capsule and/or the characteristic information of the target object.

In certain embodiments, the cooling or heating module 302 may be configured to perform a cooling or heating beauty-care operation using the control information determined based on the information from the electronic tag of the cosmetic capsule and/or the characteristic information of the target object.

In certain embodiments, the vibration module 303 may be configured to perform a beauty-care operation of vibrating massage using the control information determined based on the information from the electronic tag of the cosmetic capsule and/or the characteristic information of the target object.

In certain embodiments, the lighting module 304 may be configured to perform a beauty-care operation of illumination using the control information determined based on the information from the electronic tag of the cosmetic capsule and/or the characteristic information of the target object.

In certain embodiments, the other beauty care functional modules 305 may perform other beauty-care operations using the control information determined based on the information from the electronic tag of the cosmetic capsule and/or the characteristic information of the target object. For example, other beauty care functional modules 305 may include one or more following functional modules: a humidification module, a radio frequency module, a micro-current control module, an ultrasound module, and the like.

In certain embodiments, more specifically, the control information may include at least one of one or more of a first-type information, a second-type information, a third-type information, a fourth-type information, and a fifth-type information, etc.
(1) The first-type information may be used for controlling the beauty-care-solution-mixing control module to output the ingredients of the beauty-care-solution mixture, so that the flow of the various beauty care solutions may be controlled by the first-type information, and then the desired beauty-care-solution mixture can be made. For example, the first-type information may indicate that the ingredients of the beauty-care-solution mixture include: 20% beauty care solution A, 50% beauty care solution B, and 30% beauty care solution C, etc. Other compositions are of course as well possible.
(2) The second-type information may be used for controlling the cooling and/or heating module to perform a cooling or heating operation on the target object.
(3) The third-type information may be used for controlling the vibration module to perform a vibration operation on the target object.
(4) The fourth-type information may be used for controlling the light irradiation module to emit light on the target object.
(5) The fifth-type information may beused for controlling the other beauty care functional modules to performing other beauty-care operations related to the target object.

It can be understood that the format of the control information or the transmission mode of the control information is not limited herein.

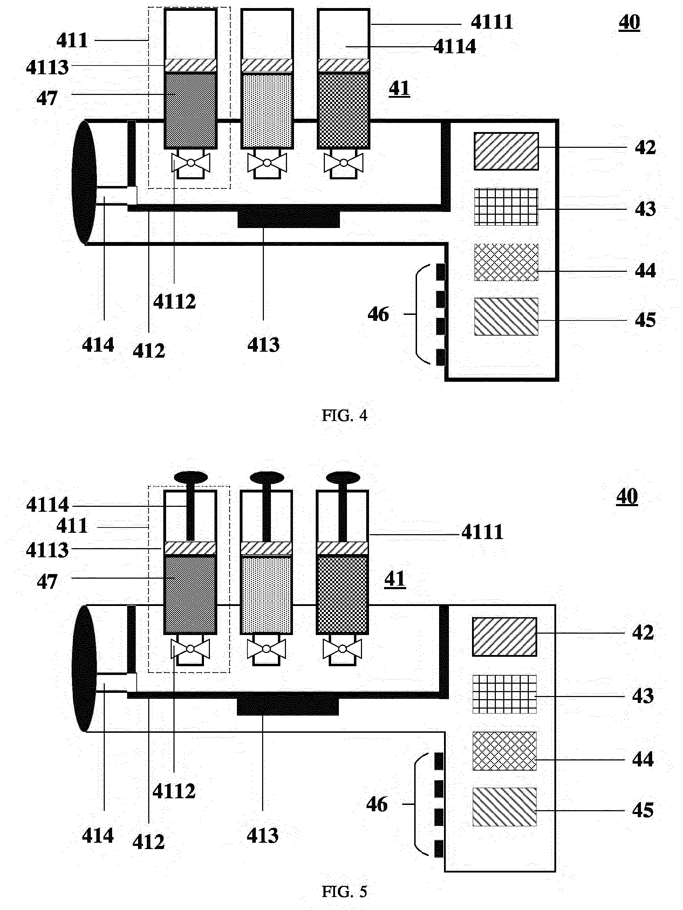

More specifically, Figures 4-6 illustrate an exemplary beauty system 40 incorporating various functional modules consistent with the disclosed embodiments of the present disclosure. Although Figures 4-6 illustrate the shape of the beauty system 40 being approximately L-shaped, which is convenient for the user to operate by hand, any appropriate shape can be used.

As shown in Figures 4-6, the beauty system 40 may include at least one of a beauty-care-solution-mixing control module 41 (or 301), a skin detection module 42, a communication module 43, a light irradiation module 44 (or 304), a radio frequency (RF) module 45, and a control switch 46. The communication module 43 may be connected to the skin detection module 42, and the skin detection module 42 may also be connected to the beauty-care-solution-mixing control module 41, the light irradiation module 44, and the radio frequency module 45, respectively. The control switch 46 can be respectively connected with at least one of the beauty-care-solution-mixing control module 41, the skin detection module 42, the communication module 43, the light-emitting module 44, and the radio frequency module 45.The optional control switch 46 may be used to switch on and off each of these functional modules. Although the cooling or heating module 302, the vibration module 303, and other beauty care functional modules 305, as shown in FIG. 3, are not shown in Figures 4-6, any one or more of these modules may be included in the beauty system 40 in Figures 4-6 similarly.

In certain embodiments, the characteristic information of the target object may include characteristic information about the skin of the target object, and the skin detection module 42 may be used to detect certain skin properties of the target object, such as skin moisture, skin temperature, skin pH value, and skin oiliness, etc. The skin detection module 42 may include one or more of a humidity detection unit, a temperature detection unit, a pH detection unit, and a skin oiliness detection unit. The humidity detection unit may include a humidity sensor (e.g., sensor 402) and a signal conditioning circuit. The humidity sensor may be a polymer membrane humidity sensitive capacitance sensor. The temperature detection unit may include a temperature sensor (e.g., sensor 402), an operational amplification circuit, and an analog to digital conversion circuit. The pH detection unit includes a pH sensor (e.g., sensor 402), an operational amplification circuit, and an analog to digital conversion circuit, and the pH sensor may be a skin-type electrode sensor. The skin oiliness detection unit may include a skin oiliness sensor (e.g., sensor 402), an operational amplification circuit, and an analog to digital conversion circuit, and the skin oiliness sensor may be a skin-type electrode sensor.

During operation, the beauty system 40 or the various sensors may make contact with a skin region of the user and may provide readings or detection results on skin moisture, skin temperature, skin pH value, and skin oiliness of the skin region to the system controller of the beauty system 40 or may be sent out through the communication module 43. The communication module 43 may include communication interfaces for wireless or wired communication, such as Cellular, Bluetooth, WiFi, or NFC.

The light irradiation module 44 may include a light emitting diode (LED) light source, a laser light source, or other light source to provide light irradiation at a light wavelength with, for example, a range of 410nm-640nm. The radio frequency module 45 may include a plurality of RF electrodes to emit radio waves at various frequencies, such as approximately 1MHz.

Further, as shown in Figures 4-6, the beauty-care-solution-mixing control module 41 may include at least one of a container portion 411 for containing a beauty-care solution (also referred to as a cosmetic fluid) 47, a collecting portion 412 for collecting the beauty-care solution 47, an agitating portion 413 for agitating the beauty-care solution(s), and a spraying portion 414 for spraying the beauty-care-solution mixture. At least a portion of the optional agitating portion 413 may be located in the collecting portion 412. The spraying portion 414 may be in communication with the collecting portion 412. The optional container portion 411 may include at least one of: a container 4111 and an actuator 4112 fixed to one end of the container 4111 and configured to control whether the beauty-care solution 47 in the container 4111 flows into the collecting portion 412.

In the example shown in FIGS. 4-6, three container portions 411 may be provided, the beauty-care solution 47 in each of the container portions 411 may be different. The number of the container portions 411 may be determined based on actual requirements. In certain embodiments, the number of the container portions 411 is one; in other embodiments, the number of the container portions 411 is two; in other embodiments, the number of the container portions 411 is three or more.

In certain embodiments, the optional collecting portion 412 may be fixed below the container portion 411. The collecting portion 412 may also be a collecting groove, and the material and shape of the collecting portion 412 are not specifically limited.

In certain embodiments, the agitating portion 413 may generate high-frequency vibration and/or magnetic rotation for various beauty-care solutions collected by the collecting portion 412, and the various beauty-care solutions may be uniformly mixed to obtain a beauty-care-solution mixture. The agitating portion 413 may be a magnetic stirrer, for example, the magnetic stirrer may include: a stirring table and a magnet.

In certain embodiments, the spray portion 414 may be secured to one side of the collecting portion 412, and is a liquid atomization device or a micro-electric vacuum motor that is coupled to the collecting portion 412 by a conduit.

In certain embodiments, the material of the container 4111 may include at least one of, but is not limited to, glass, plastic, metal, etc., the container 4111 is resistant to high pressure and does not contaminate the beauty-care solution, and the shape and material of the container 4111 are not specifically limited.

In certain embodiments, the optional actuator 4112 may be an electronically controllable switch, including but not limited to a solenoid valve. The actuator 4112 may include a conductive state (ON state) and a closed state (OFF state). When the actuator 4112 is in the conductive state, the beauty-care solution can flow into the collecting portion 412; when the actuator 4112 is in the closed state, the beauty-care solution cannot flow into the collecting portion 412.

The beauty-care-solution-mixing control module may be controlled based on the first-type information to output the ingredients of the beauty-care-solution mixture. In certain embodiments, the first-type information may include one or more of various parameters for the optional beauty-care-solution-mixing control module.

For example, the first-type information may include a first parameter for controlling one or more actuators 4112 to permit and/or inhibit the flow of beauty-care solution 47 in the container 4111 into the collection portion 412. The first parameter may instruct that the actuator 4112 is to be activated, and the time duration during which the actuator 4112 is in the conductive or ON state. For example, when having three containers (container A, container B and container C) and three actuators (actuator A, actuator B and actuator C), the first parameter may instruct: the actuator A to be activated, and the time duration during which the actuator A is in the ON state; the actuator B to be activated, and the time duration during which the actuator B is in the ON state; the actuator C to be activated, and the time duration during which the actuator C is in the ON state. Thus, the beauty-care solutions in the container A, the container B, and the container C can flow into the collecting portion 412, and the amount of each beauty-care solution can be controlled by controlling the time duration of the ON state of each actuator.

The first-type information may include a second parameter for controlling the agitating portion 413 to agitate the beauty-care solution mixture for example in the optional collecting portion 412. For example, the second parameter instructs that the magnetic stirrer is to be activated, and the time duration during which the magnetic stirrer is operational.

Further, the first-type information may include a third parameter used to control the spraying portion 414 to spray the beauty-care-solution mixture. For example, the third parameter may instruct that the atomizing nozzle to be activated, and the particle size for the atomization.

The beauty-care solution mixing control module 41 may be implemented in various ways and structures. In certain embodiments, as shown in FIG. 4, the beauty-care solution mixing control module 41 may further include a piston 4113 and a high-pressure gas 4114 disposed in the container 4111. The high-pressure gas 4114 may be located on one side of the piston 4113. The beauty-care solution 47 may be located on the other side of the piston 4113. When the pressure on the side of the high-pressure gas 4114 is greater than the pressure on the side of the beauty-care solution 47, the high-pressure gas 4114 can push the piston 4113 toward the beauty-care solution 47 side, thereby push the beauty-care solution 47 for example into the optional collecting portion 412.

Further, the first parameter may be used to control the optional actuator 4112 to allow the beauty-care solution in the container 4111 to flow e.g. into the optional collecting portion 412. For example, the first parameter may include: information instructing that the actuator 4112 is to be in the ON state, and the time duration for the ON state.

It should be noted that the function of the high-pressure gas 4114 may be to enable the beauty-care solution in the container 4111 to flow into the collecting portion 412 by the pressure difference. Any appropriate pressure values and high-pressure gases can be used.

In certain embodiments, when using the beauty system shown in FIG. 4, the terminal may acquire the result of the skin detection, and the environmental information (for example, the current location information and the climate information) through an application program (APP), and may obtain the control information for beauty care based on the skin detection result and the environmental information, and send the control information to the beauty system. After the beauty system receives the control information through the communication module 43, the actuator 4112 may be instructed to be in an ON state, and the high-pressure gas 4114 in the container 4111 may pushe the piston 4113 under the pressure difference, and then the beauty-care solution 47 is squeezed e.g. into the optional collecting portion 412. Alternatively, the time duration during which the actuator 4112 is in the ON state is adjusted according to the magnitude of the air pressure difference. Thus, the amount of the beauty-care solution 47 flowing out and into the colleting portion 412 can be accurately controlled. The plurality of optional containers 4111 can be individually loaded into the beauty-care solution 47 of different compositions as needed. The amount of the beauty-care solution 47 in each container 4111 may be separately controlled in accordance with the control information at the time of use.

The various beauty-care solutions 47 may be uniformly introduced into the optional collecting portion 412, and after the beauty-care solution 47 is collected, the agitating portion 413 may generate high-frequency vibration and/or magnetic rotation, and the various beauty-care solutions 47 may be uniformly mixed and sprayed by the spraying portion 414.

In certain embodiments, referring to FIG. 5, the beauty-care-solution-mixing control module 41 may further include at least one of: a piston 4113 disposed within the container 411, and a driving portion 4114 for driving the piston to move within the container. One end of the driving portion 4114 may protrude into the container 411 and may be connected to the piston 4113. Optionally, the driving portion 4114 may include a screw motor.

Further, the first parameter is used to control the actuator 4112 to allow the beauty-care solution in the container 4111 to flow e.g. into the optional collecting portion 412. For example, the first parameter may include: information instructing that the actuator 4112 is to be in the ON state, and the time duration of the ON state. The control information may further include a fourth parameter, which is used to control the driving portion 4114 to drive the piston 4113 to move within the container 411. When the driving portion 4114 is a screw motor, the fourth parameter is used to control the motor rotation speed of the screw motor.

In certain embodiments, shown in FIG. 5, when the beauty system is used, the terminal may acquire the skin detection result and the environmental information (for example, the current location information and the climate information) through the APP, and the terminal may obtain the control information for beauty care based on the skin detection result and the environmental information and may send the control information to the beauty system. After the beauty system receives the control information through the communication module 43, the actuator 4112 may be instructed to be in an ON state, the screw motor in the container 4111 may pushe the piston 4113 to move, and then the beauty-care solution 47 may be squeezed into the collecting portion 412. Alternatively, the amount of the beauty-care solution 47 flowing out can be precisely controlled according to the time and flow rate at which the screw motor operates. The plurality of containers 4111 can be individually loaded in the beauty-care solution 47 of different compositions as needed. The amount of the beauty-care solution 47 in each container 4111 may be separately controlled in accordance with the control information at the time of use.

The various beauty-care solutions 47 may be uniformly introduced into the collecting portion 412, and after the beauty-care solutions 47 may be collected, the agitating portion 413 may generate high-frequency vibration and/or magnetic rotation, and the various beauty-care solutions 47 may be uniformly mixed and optionally sprayed by the spraying portion 414.

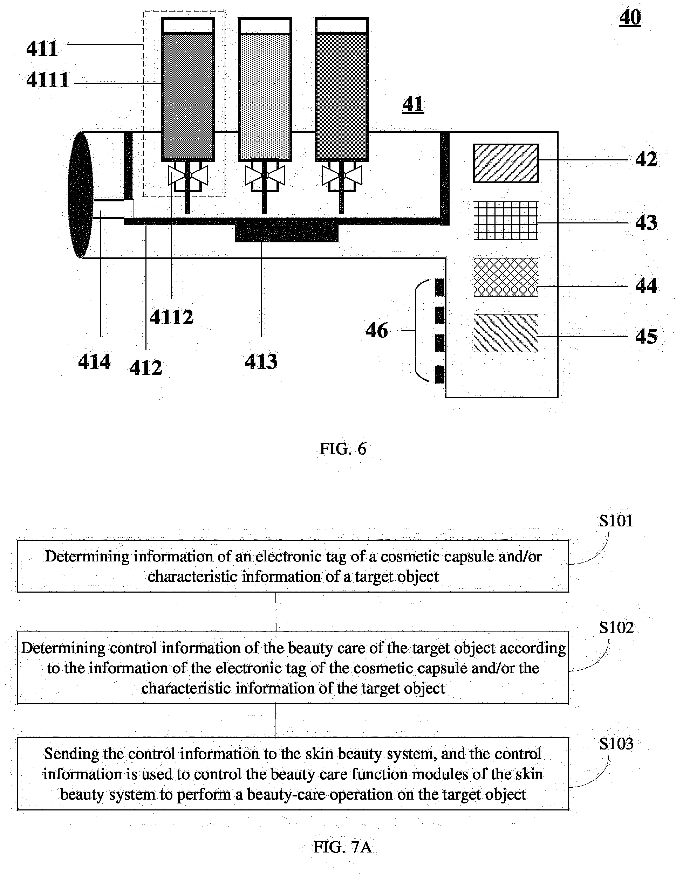

In certain embodiments, referring to FIG. 6, actuator 4112 may be a micropump, and the first parameter may include a flow parameter and/or a power parameter of the micropump.

In certain embodiments, when the beauty system shown in FIG. 6 is used, the terminal may acquire at least one of the skin detection result and the environmental information (for example, including the current location information and the climate information) through the APP, and the terminal may obtain the control information for the beauty care based on at least one of the the skin detection result and the environmental information, and may send the control information to the beauty system. The beauty system may instruct the micropump to operate after receiving the control information through the communication module 43, and the beauty-care solution 47 in the container 4111 may flow into the collecting portion 412 by the action of the micropump. Alternatively, the time duration of the operation can be controlled according to the flow rate and power parameters of the micropump, and precise control can be performed to accurately control the outflow of the beauty-care solution 47. The plurality of containers 4111 can be individually loaded in the beauty-care solution 47 of different compositions as needed. The amount of the beauty-care solution 47 in each container 4111 may be separately controlled in accordance with the control information at the time of use.

The various beauty-care solutions 47 may be uniformly introduced into the collecting portion 412, and after the beauty-care solutions 47 may be collected, the agitating portion 413 may generate high-frequency vibration and/or magnetic rotation, and the various beauty-care solutions 47 may be uniformly mixed and preferably sprayed by the spraying portion 414.

Accordingly, the beauty system may perform the corresponding beauty-care operation according to the control information determined by the terminal, instead of setting the beauty-care operation uniformly by the beauty system, so that the beauty-care mode can be different and targeted for different users, effectively improving the beauty treatment effect.

Returning to FIG. 1, during operation, the beauty system 40 and the terminal 10 may perform certain beauty-care methods to perform beauty care on a user of the terminal 10 or beauty system 40. FIG. 7A illustrates an exemplary beauty care method for the terminal 10 according to certain embodiments of the present disclosure. As shown in FIG. 7A, the beauty care method may include at least one of the followings.

S101: Determining information of an electronic tag of a cosmetic capsule and/or characteristic information of a target object.

In certain embodiments, the cosmetic capsule may be a health product for beauty care. It is to be understood that the beauty capsule is not specifically limited, and may include any appropriate health product.

In certain embodiments, the corresponding beauty care method (such as the composition of the beauty-care-solution mixture, the method of use, the manufacturer, the date, and the like) may be recorded in the electronic tag of the cosmetic capsule. The information can be determined by the terminal or the beauty system for identification. On the one hand, the information can be used to identify the authenticity and shelf-life of the health product, and on the other hand, it can be used in conjunction with the corresponding beauty system to better perform the beauty care. Optionally, the electronic tag of the cosmetic capsule is a radio frequency tag, such as a radio frequency identification (RFID) tag, or an NFC tag.

In certain embodiments, the target subject may be a human a plant or an apparatus or the skin of a human, a plant or a surface of an apparatus. In certain embodiments, the target subject is an animal or the skin of the animal. It can be understood that the type of the target object is not limited in the present disclosure.

In certain embodiments, the characteristic information of the target object may include one or more of: (1) a skin detection result of the target object, for example, the skin detection result may include information on one or more of: skin age, skin color, and skin oiliness, spots, blackheads, pores, smoothness, wrinkles, moisture, acne, texture, etc.; (2) time information to perform the beauty care operation on the target object, for example, a certain day of the year, morning, noon, afternoon, or evening; (3) geographic information of the location of the target object, for example, latitude and longitude information of the current location; (4) environment information of the location of the target object, for example, current climate information, which may include the temperature and/or humidity.

S102: Determining control information of the beauty care operation of the target object according to the information of the electronic tag of the cosmetic capsule and/or the characteristic information of the target object.

In certain embodiments, the control information of the beauty care operation of the beauty system on the target object may be determined based on the information of the electronic tag of the cosmetic capsule and/or the characteristic information of the target object.

S103: Sending the control information to the beauty system, and the control informationmay be used to control the beauty care function modules of the beauty system to perform the beauty-care operation on the target object.

In certain embodiments, the beauty-care operation may include, but is not limited to, one or more of: spraying of the beauty-care-solution mixture, vibrating, light irradiating, cooling or heating, and other care operations, etc. Alternatively, other care operations include one or more of: electrical muscle stimulation (EMS), radio frequency, ultra pulse puncture, mild frequency interval pulse (MFIP), and the like.

In certain embodiments, according to the information of the electronic tag of the cosmetic capsule, a beauty care mode matching the cosmetic capsule may be determined. The beauty mode may refer to specific steps of beauty care, a specific beauty care method, specific steps of handling a health product, specific steps of handling ingredients, or other similar combinations. Further, according to the beauty care mode and characteristic information of the target object, the control information for the beauty care operation on the target object may be determined. Optionally, the correspondence between the control information of the beauty care operation and the beauty care mode and the characteristic information of the target object may be pre-configured.

In certain embodiments, the beauty care mode that matches the cosmetic capsule refers to one or more beauty care modes recommended by the cosmetic capsule, and desired beauty care effect may be achieved when the one or more beauty care modes may be used with the cosmetic capsule. For example, the beauty capsule 1 recommends the beauty care mode 1 and the beauty care mode 2; the beauty care mode 2 may be selected according to the characteristic information of the target object; and then the beauty care control information of the target object may be determined according to the beauty care mode 2. Optionally, the correspondence between the characteristic information and the beauty care mode may be pre-configured, and the correspondence between the beauty care mode and the control information may be also pre-configured.

In certain embodiments, in S101, the information of the electronic tag of the cosmetic capsule and/or the characteristic information of the target object may be acquired by a specific module installed on the terminal, and the above module may be implemented by software (for example, APP) or hardware. For example, an electronic tag reader or an electronic tag recognition module may be used to obtain information of the electronic tag of the cosmetic capsule, a skin detection module (e.g., a skin detecting APP) may be used to obtain skin detection result of the target object; a time module (e.g., a time APP) may be used to acquire time information; a positioning module (e.g., a positioning APP) may be used to obtain geographic information; and a weather module (e.g., a weather APP) may be used to obtain to environment information.

In certain embodiments, in S101, the terminal receives information of the electronic tag of the cosmetic capsule and/or characteristic information of the target object from the beauty system. For example, at least one of the electronic tag recognition module, the skin detection module, the time module, positioning module, weather module, and wireless transmission module, etc., may be installed on the beauty system.

It can be understood that the positioning module and the weather module can be installed on the beauty system or can be installed on the terminal. I n certain embodiments, the positioning module and the weather module can be installed on the terminal in association with the APP on the terminal, which can save both the equipment cost of the beauty system and the size of the beauty system.

In certain embodiments, before S101, the correspondence between the control information of the beauty care operation and one or more of the information of the electronic tag of the cosmetic capsule, the skin detection result, the time information, the geographic information, and the environment information can be pre-configured.

**Table 1: Correspondence between the control information of beauty care operation and one or more of the electronic tag information of beauty capsules, skin detection results, time information, geographic information, and environment information.**

| | Skin detection result | Time information | Environment information | Electronic tag information of beauty capsule | control information of the beauty care operation |
|---|---|---|---|---|---|
| 1 | A1 | B1 | C1 | E1 | D1 |
| 2 | A2 | B2 | C2 | E2 | D2 |
| 3 | A3 | B3 | C3 | E3 | D3 |

In Table 1, the correspondence is established between the control information of the beauty care operation D1 and one or more of skin detection result A1, time information B1, geographic information C1, and environment information E1. The correspondence is established between the control information of the beauty care operation D2 and one or more of skin detection result A2, time information B2, geographic information C2, and environment information E2. The correspondence is established between the control information of the beauty care operation D3 and one or more of skin detection result A3, time information B3, geographic information C3, and environment information E3. It can be understood that A1∼A3, B1∼B3, C1∼C3, D1∼D3, and E1∼E3 are not limited to specific contents.

In certain embodiments, the information of the electronic tag of the cosmetic capsule may include: a beauty care mode matched with the cosmetic capsule, and the beauty care mode matched with the cosmetic capsule may include one or more of: a composition of the beauty-care-solution mixture, a method using the beauty-care-solution mixture, etc.

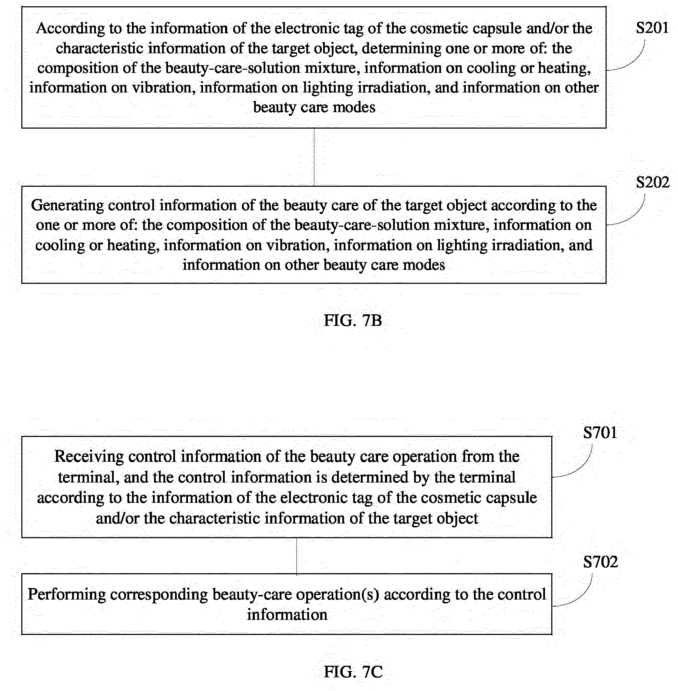

In certain embodiments, referring to FIG. 7B, the process of determining, according to the electronic tag of the cosmetic capsule and/or characteristic information of the target object, the control information of the beauty care of the target object may include the followings.

S1011: According to the information of the electronic tag of the cosmetic capsule and/or the characteristic information of the target object, determining one or more of: the composition of the beauty-care-solution mixture (the types of the beauty-care solutions contained in the beauty-care-solution mixture and the quantity of each beauty-care solution), information on cooling or heating, information on vibration, information on lighting irradiation, and information on other beauty care modes.

S1012: Generating control information of the beauty care of the target object according to the one or more of: the composition of the beauty-care-solution mixture, information on cooling or heating, information on vibration, information on lighting irradiation, and information on other beauty care modes.

In certain embodiments, the beauty-care-solution mixture contains at least two beauty-care solutions. For example, the beauty-care solutions may be at least two essence solutions, such as a plant essence solution, a fruit-acid essence solution, an animal essence solution, a vitamin essence solution, a genetic essence solution, or the like.

**Table 2: Correspondence between the information of the electronic tag of the cosmetic capsule, the characteristic information of the target object, the composition of the beauty-care-solution mixture, the cooling or heating information, the vibration information, the light irradiation information, and the information of other beauty care modes.**

| | electronic tag information of the cosmetic capsule | characteristic information of the target object | composition of the beauty-care-solution mixture | cooling or heating information | vibration information | light irradiation information | other beauty care modes |
|---|---|---|---|---|---|---|---|
| 1 | K1 | E1 | F1 | G1 | H1 | I1 | J1 |
| 2 | K2 | E2 | F2 | G2 | H2 | I2 | J2 |

In Table 2, the correspondence is established between the information of the electronic tag of the cosmetic capsule K1 and/or the characteristic information of the target object E1 and the composition of the beauty-care-solution mixture F1, the cooling or heating information G1, the vibration information H1, the light irradiation information 11, and the information of other beauty care modes J1. Further, the correspondence is established between the information of the electronic tag of the cosmetic capsule K2 and/or the characteristic information of the target object E2 and the composition of the beauty-care-solution mixture F2, the cooling or heating information G2, the vibration information H2, the light irradiation information 12, and the information of other beauty care modes J2. It can be understood that E1∼E2, F1∼F2, G1∼G2, H1∼H2, I1∼12, J1∼J2, K1∼K2 are not limited to any specific contents.

In certain embodiments, the beauty-care-solution mixture may be produced according to the ingredients of the beauty-care-solution mixture. For example, the following information may be recorded in F1: the beauty-care-solution mixture A may include a beauty-care solution 1 and a beauty-care solution 2, the quantity of the beauty-care solution 1 is a, and the quantity of the beauty-care solution 2 is b. Further, the following information may be recorded in F2: the beauty-care-solution mixture B may include a beauty-care solution 1, a beauty-care solution 2, and a beauty-care solution 3; the quantity of the beauty-care solution 1 is x, the quantity of the beauty-care solution 2 is y, and the quantity of beauty-care solution 3 is z.

In certain embodiments, the vibration information may include a vibration frequency. The light irradiation information may include the wavelength of the light, and the wavelength of the light may be in a range of 410 nanometers (nm) to 640 nm.

In certain embodiments, the information of other beauty care modes may include one or more of the following: spraying information, ultrasonic information, radio frequency information, micro current information, pulse information, etc. The spraying information may include: the flow rate of the spray nozzle, and the size of the atomization particles, etc.; the ultrasonic information may include the frequency of the ultrasonic wave; the radio frequency information may include the RF frequency, such as 1 MHz.

**Table 3: Correspondence between the control information and the composition of the beauty-care-solution mixture, cooling or heating information, vibration information, light irradiation information, and/or other beauty care mode information.**

| | composition of the beauty-care-solution mixture | cooling or heating information | vibration information | light irradiation information | other beauty care mode information | control information |
|---|---|---|---|---|---|---|
| 1 | F1 | G1 | H1 | I1 | J1 | L1 |
| 2 | F2 | G2 | H2 | I2 | J2 | L2 |

In Table 3, the correspondence is established between the control information L1 and the composition of the beauty-care-solution mixture F1, the cooling or heating information G1, the vibration information H1, the light irradiation information I1, and the information of other beauty care modes J1. Further, the correspondence is established between the control information L2 and the composition of the beauty-care-solution mixture F2, the cooling or heating information G2, the vibration information H2, the light irradiation information 12, and the information of other beauty care modes J2. It can be understood that L1∼L2, F1∼F2, G1∼G2, H1∼H2, I1∼I2, J1∼J2 are not limited to the specific contents.

FIG. 7C illustrates a skin beauty care method for a beauty system consistent with the embodiments of the present disclosure. As shown in FIG. 7C, the skin beauty care method may include the followings.

S701: Receiving control information of the beauty care operation from the terminal, and the control information may be determined by the terminal according to the information of the electronic tag of the cosmetic capsule and/or the characteristic information of the target object.

S702: Performing corresponding beauty-care operation(s) according to the control information.

In certain embodiments, in S702, the composition of the beauty-care solution mixture may be determined according to the control information; the beauty-care-solution mixture may be generated according to the composition of the beauty-care solution mixture; and the beauty-care-solution mixture may be sprayed to the target object.

In certain embodiments, the method further comprises: determining information of the electronic tag of the cosmetic capsule and/or characteristic information of the target object, and transmitting the information of the electronic tag of the cosmetic capsule and/or the characteristic information of the target object to the terminal.

Accordingly, the beauty system performs the corresponding beauty-care operation according to the control information determined by the terminal, instead of setting the beauty-care operation universally by the beauty system. Thus, the beauty care modes can be adjusted differently and targeted different measures for different users, effectively improving the beauty treatment effect of the beauty system.

The certain embodiments, referring to FIG. 4 to FIG. 6, the beauty system may include a beauty-care-solution-mixing control module 41, and the beauty-care-solution-mixing control module 41 may include at least one container portion 411, a collecting portion 412 for collecting the beauty-care solution, an agitating portion 413 for stirring the beauty-care solution, and a spraying portion 414 for spraying the beauty-care-solution mixture. Further, at least a portion of the agitating portion 413 may be located in the collecting portion 412, and the spraying portion 414 may be in communication with the collecting portion 412.

The container portion 411 may include a container 4111 for accommodating the beauty-care solution and an actuator 4112 fixed at one end of the container 4111, and the actuator 4112 may be configured to control whether the beauty-care solution in the container 4111 flows into the collecting portion 412.

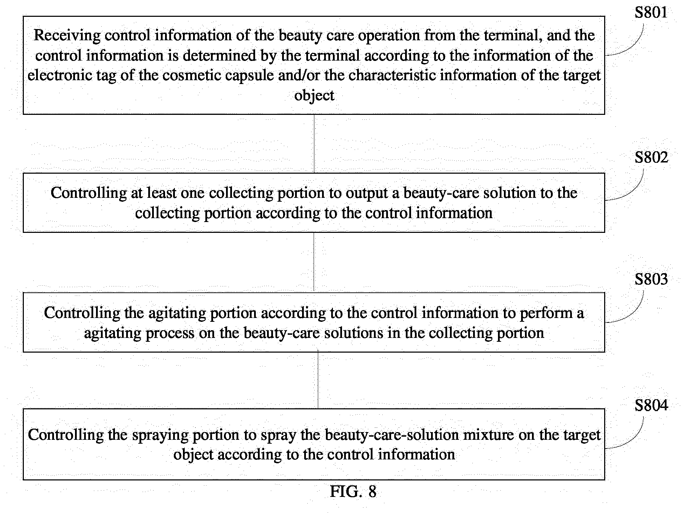

With such configuration, FIG. 8 illustrates another skin beauty care method for the beauty system consistent with the embodiments of the present disclosure. As shown in FIG. 8, the skin beauty care method may include the followings.

S801: Receiving control information of the beauty care operation from the terminal, and the control information may be determined by the terminal according to the information of the electronic tag of the cosmetic capsule and/or the characteristic information of the target object.

S802: Controlling at least one collecting portion to output a beauty-care solution to the collecting portion according to the control information.

S803: Controlling the agitating portion according to the control information to perform a agitating process on the beauty-care solutions in the collecting portion.

S804: Controlling the spraying portion to spray the beauty-care-solution mixture on the target object according to the control information.

In certain embodiments, referring to FIG. 4, the beauty-care solution mixing control module 41 may further include a piston 4113 and a high-pressure gas 4114 disposed in the container 4111, and the high-pressure gas 4114 may be located at one of the pistons 4113, while the beauty-care solution 47 is located on the other side of the piston 4113. When the pressure on the side of the high-pressure gas 4114 may be greater than the pressure on the side of the beauty-care solution 47, the high-pressure gas 4114 can push the piston 4113 toward the beauty-care solution 47 side. Thus, the beauty-care solution 47 can be push into the container 4111.

With such configuration, referring to FIG. 8, the above S802 may include: controlling the conduction actuator of the at least one container portion according to the control information, so that the beauty-care solution in the container flows into the collecting portion; and, according to the change of the pressure of the high-pressure gas in the container and/or the flow rate of the beauty-care solution flowing out of the container, controlling the time duration of the conduction of the actuator.

In certain embodiments, referring to FIG. 5, the beauty-care solution mixing control module 41 may further include a piston 4113 disposed within the container 4111, and a driving portion for driving the piston to move within the container. 4114. One end of the driving portion 4114 extends into the container 4111 and may be connected to the piston 4113.

With such configuration, the above S802 may include: controlling the conduction of the at least one container portion according to the control information, and controlling the driving portion of the at least one container portion to drive the piston movement; and, according to the flow rate of the beauty-care solution flowing out of the container to control the running time of the driving portion.

In certain embodiments, referring to FIG. 6, the actuator 4112 may be a micropump. The above S802 may include controlling the flow rate and/or power of the micropump of the at least one container portion according to the control information such that the at least one container portion outputs the beauty-care solution to the collecting portion.

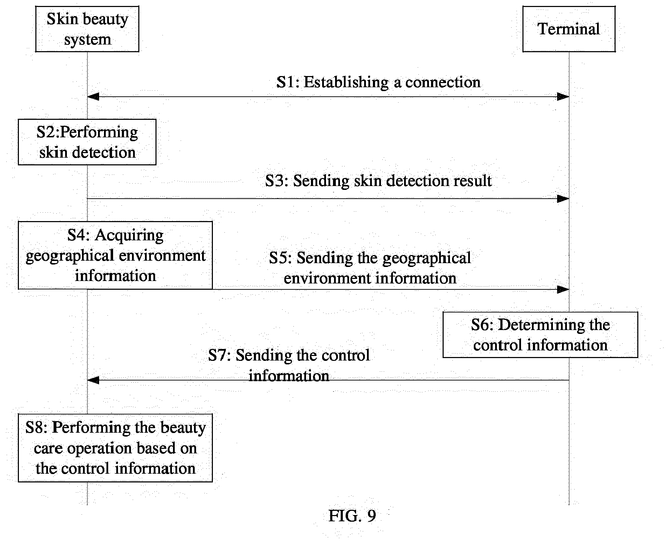

FIGS. 9-12 illustrate certain skin beauty care methods for a terminal and a beauty system consistent with embodiments of the present disclosure. Referring to FIG. 9, a skin beauty care method may include the followings.

S1: Establishing a connection between the beauty system and the terminal. The terminal may initiate the connection by the user using the APP, or the beauty system may initiate the connection, which may activate the APP on the terminal to prompt the user.

S2: The beauty system performs skin detection on the target object.

S3: The beauty system sends the skin detection result to the terminal.

S4: The beauty system acquires geographic environment information. For example, the geographic environment information may include: geographic information and environment information.

S5: The beauty system sends the geographic environment information to the terminal.

S6: The terminal determines the control information of the beauty care operation of the target object according to the skin detection result and the geographic environment information.

S7: The terminal sends the control information to the beauty system;

S8: The beauty system performs the corresponding beauty-care operation according to the control information.

Other steps may also be included. It can be understood that, in certain embodiments, the above steps may be performed in sequence or out of sequence show above.

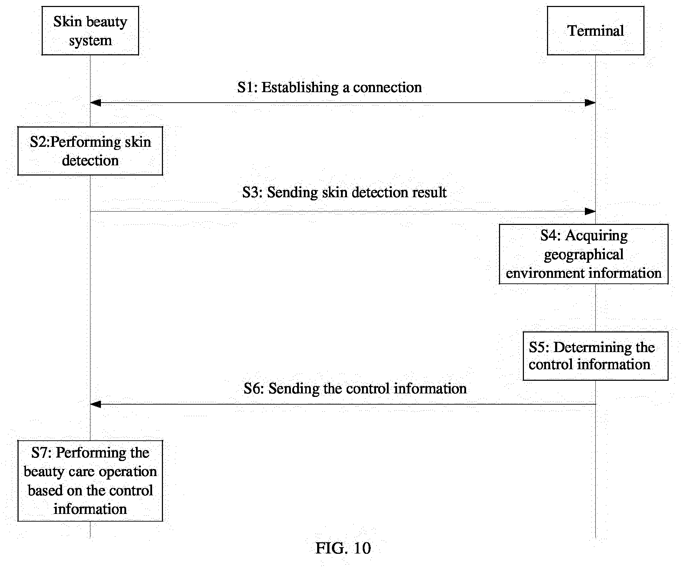

Referring to FIG. 10, another skin beauty care method may include the followings.

S1: Establishing a connection between the beauty system and the terminal.

S2: The beauty system performs skin detection on the target object.

S3: The beauty system sends the skin detection result to the terminal.

S4: The terminal acquires geographic environment information.

S5: The terminal determines the control information of the beauty care operation of the target object according to the skin detection result and the geographic environment information.

S6: The terminal sends the control information to the beauty system.

S7: The beauty system performs the corresponding beauty-care operation according to the control information.

Other steps may also be included. It can be understood that, in certain embodiments, the above steps may be performed in sequence or out of sequence show above.

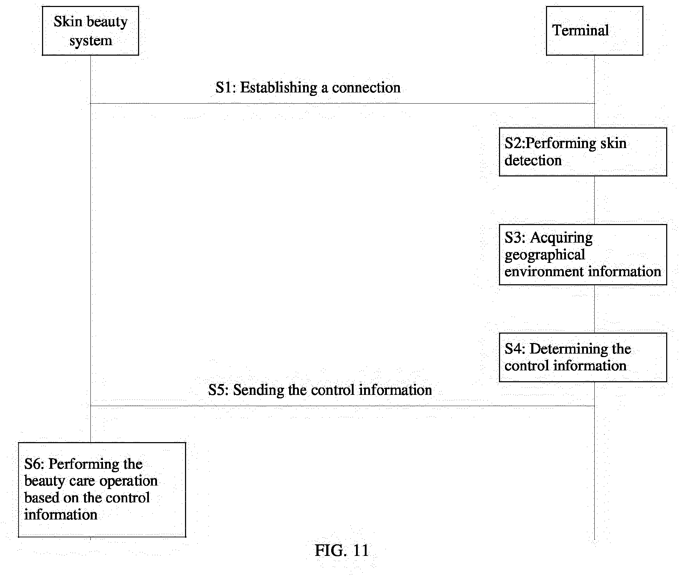

Referring to FIG. 11, another skin beauty care method may include the followings.

S1: Establishing a connection between the beauty system and the terminal.

S2: The terminal performs skin detection on the target object.

S3: The terminal acquires geographic environment information.

S4: The terminal determines the control information of the beauty care operation of the target object according to the skin detection result and the geographic environment information.

S5: The terminal sends the control information to the beauty system.

S6: The beauty system performs the corresponding beauty-care operation according to the control information.

Other steps may also be included. It can be understood that, in certain embodiments, the above steps may be performed in sequence or out of sequence show above.

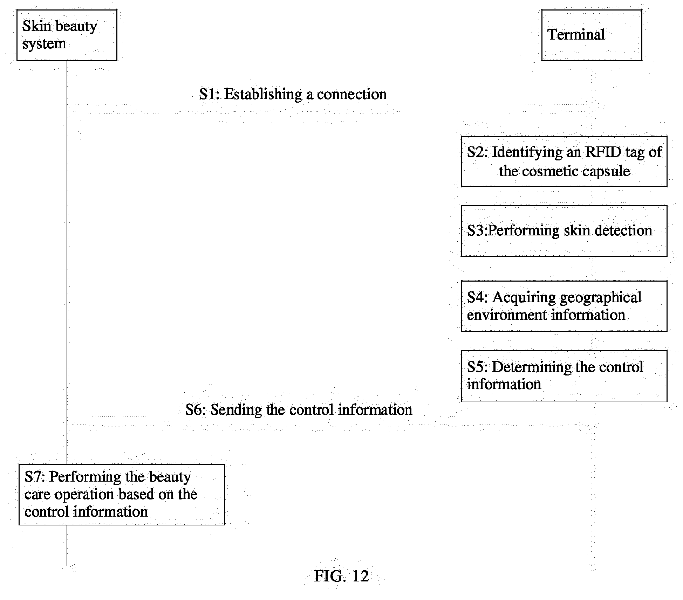

Referring to FIG. 12, another skin beauty care method may include the followings.

S1: Establishing a connection between the beauty system and the terminal.

S2: The terminal identifies an RFID tag of the cosmetic capsule.

S3: The terminal performs skin detection on the target object.

S4: The terminal acquires geographic environment information.

S5: The terminal determines the control information of the beauty care operation of the target object according to the information of the RFID tag of the cosmetic capsule, the skin detection result, and the geographic environment information, etc.

S6: The terminal sends the control information to the beauty system.

S7: The beauty system performs the corresponding beauty-care operation according to the control information.

Other steps may also be included. It can be understood that, in certain embodiments, the above steps may be performed in sequence or out of sequence show above.

In certain embodiments, an RFID tag may be integrated into the cosmetic capsule, and the corresponding composition of the beauty-care-solution mixture, the method of use, the manufacturer, and the date can be recorded in the RFID tag for identification by the terminal. Such information can be used to identify the authenticity, expiration date, etc., of the product, and can also be used in conjunction with the corresponding beauty system to achieve better beauty care effect.

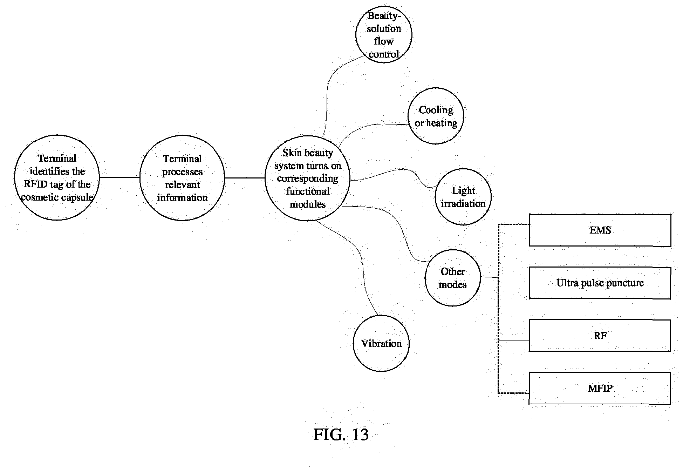

FIG. 13 illustrates an exemplary operation process based on a beauty capsule integrated with the electronic tag. As shown in FIG. 13, the beauty capsule integrated with the electronic tag may be identified by the terminal, and then it may be determined whether the beauty capsule can be used (for example, whether it is a company-specific product, whether it is within the warranty period, etc.), and then it can be determined what kind of beauty care modes are recommended for using the beauty capsule. Further, combined with other relevant information obtained by the terminal (such as environment temperature, environment humidity, skin detection results, etc.), the terminal informs the beauty system to turn on the corresponding functional modules (such as heating or cooling, vibrating, light irradiation, beauty-care solution flow control, usage of micro-current or RF or ultra-pulse puncture, etc.). Thus, better beauty care effect may be achieved.

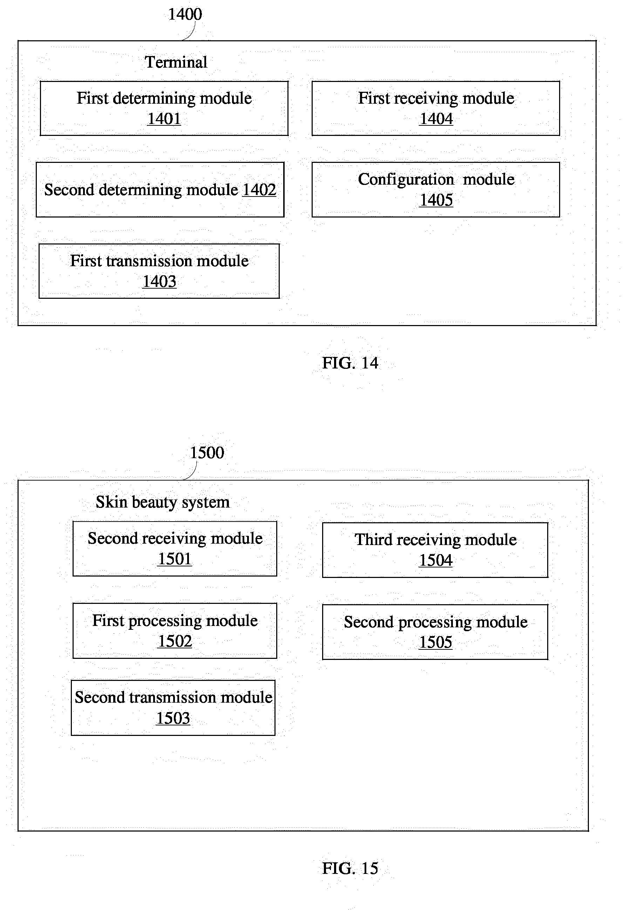

FIG. 14 illustrates an exemplary block diagram of a terminal consistent with the embodiments of the present disclosure. As shown in FIG. 14, terminal 1400 may include a first determining module 1401, a second determining module 1402, and a first transmission module 1403. Other modules may also be included.

The first determining module 1401 may be configured to determine information of the electronic tag of the cosmetic capsule and/or characteristic information of the target object. The second determining module 1402 may be configured to determine, according to the information of the electronic tag of the cosmetic capsule and/or characteristic information of the target object, control information of the beauty care operation of the target object.

The first transmission module 1403 may be configured to send the control information to the beauty system, where the control information may be used to control the beauty system to perform a corresponding beauty care operation on the target object.

In some embodiments, the second determining module 1402 may be further configured to: determine a desired beauty care mode matching the cosmetic capsule according to the information of the electronic tag of the cosmetic capsule; and, according to the beauty care mode and the characteristic information of the target object, determine the control information of the beauty care operation of the target object.

In some embodiments, the terminal 1400 may further include: a first receiving module 1404, which may be configured to receive the information of the electronic tag of the cosmetic capsule and/or the characteristic information of the target object from the beauty system.

In some embodiments, the characteristic information of the target object may include one or more of: a skin detection result of the target object; time information for performing beauty care on the target object; geographic information of the location of the target object; and the environment information of the location of the target object, etc.

In some embodiments, the terminal 1400 may further include: a configuration module 1405, which may be configured to pre-configure a correspondence relationship between at least one of: the control information of the beauty care and the information of the electronic tag of the cosmetic capsule, the skin detection result, the time information, geographic information, and the environment information.

In some embodiments, the second determining module 1402 may be further configured to: determine a composition of the beauty-care solutions included in the beauty-care-solution mixture according to the information of the electronic tag of the cosmetic capsule and/or the characteristic information of the target object, and to generate control information of the beauty care of the target object based on the composition of the beauty-care solutions contained in the beauty-care-solution mixture.

FIG. 15 illustrates an exemplary block diagram of a beauty system consistent with the embodiments of the present disclosure. As shown in FIG. 15, the beauty system 1500 may include a second receiving module 1501, and a processing module 1502, etc.

The second receiving module 1501 may be configured to receive from the terminal the control information of the beauty care on the target object. The control information may be determined by the terminal according to information of the electronic tag of the cosmetic capsule and/or characteristic information of the target object. The processing module 1502 may be configured to perform a corresponding beauty care operation on the target object according to the control information.

In some embodiments, the beauty system 1500 may further include: a second transmission module 1503, which may be configured to determine the information of the electronic tag of the cosmetic capsule and/or the characteristic information of the target object, and to send the information of the electronic tag of the cosmetic capsule and/or the characteristic information of the target object.

In some embodiments, the second processing module 1502 may be further configured to: determine a composition of the beauty-care solutions included in the beauty-care-solution mixture according to the control information; to generate the beauty-care-solution mixture based on the composition of the beauty-care solutions; and to spray the beauty-care-solution mixture on the target object.

Further, the beauty system 1500 may further include a third receiving module 1504 and a second processing module 1505, etc. The third receiving module 1504 may be configured to receive the control information of the beauty care from the terminal, and the control information may be determined by the terminal according to the information of the electronic tag of the cosmetic capsule and/or the characteristic information of the target object.

The second processing module 1505 may be configured to control, according to the control information, at least one container portion of the beauty system to output a beauty care solution to the collecting portion of the beauty system; to control, according to the control information, the agitating portion of the beauty system to agitate the beauty care solutions in the collecting portion; and to control, according to the control information, the spraying portion of the beauty system to spray the beauty-care-solution mixture on the target object.

Various embodiments of the present specification are described in a progressive manner, each embodiment may have its difference from other embodiments, and features of different embodiments may be combined together in any appropriate manner or may be separated from each other.

The above specification that discloses various embodiments in intended for those skilled in the art to practice or use this disclosure. Various modifications of these embodiments are apparent to those skilled in the art, and the basic principles defined in this paper can be realized in other embodiments without departing from the spirit or scope of this disclosure. As such, this disclosure will not be limited to the disclosed embodiments, but rather it is intended to satisfy the widest range that is consistent with the principles and novel ideas made common by this disclosure.

## Claims

1. A beauty care system (40), comprising:
a communication module (43) for establishing a communication link (20) with a user terminal (10); and
a beauty-care-solution-mixing control module (301) for receiving control information from the user terminal (10) over the communication link (10) and performing a beauty-care operation on a target object based on the control information from the user terminal (10),
**characterized in that** the control information is determined based on at least one of information of an electronic tag of a cosmetic capsule and characteristic information of the target object.

2. The beauty care system (40) according to claim 1, **characterized in that** the beauty-care-solution-mixing control module (301) includes:
one or more container portions (411) each contain a beauty-care solution;
a collecting portion (412) coupled to the container portions (411) to collect the beauty-care solutions outputted from the container portions (411);
an agitating portion (413) coupled to the collecting portion (412) for agitating the beauty-care solutions in the collecting portion (412) to form a beauty-care-solution mixture; and
a spraying portion (414) for spraying the beauty-care-solution mixture on the target object.

3. The beauty care system (40) according to claim 2, **characterized in that** each container portion (411) includes:
a container (4111) configured to contain the beauty-care solution; and
an actuator (404, 4112) fixed to one end of the container (4111) and configured to control the beauty-care solution outputted into the collecting portion (412).

4. The beauty care system (40) according to claim 3, **characterized in that**:
the control information include a first-type information used for controlling the beauty-care-solution-mixing control module (301), such that flows of the beauty-care solutions are controlled to output desired ingredients of the beauty-care-solution mixture; and
the first-type information includes:
a first parameter for controlling each actuator (404, 4112) to the flow of the beauty-care solution in the container (4111) into the collection portion (412) by instructing whether the actuator (404, 4112) is to be activated and a time duration during which the actuator (404, 4112) is activated;
a second parameter for controlling the agitating portion (413) to agitate the beauty-care solutions in the collecting portion (412); and
a third parameter for controlling the spraying portion (414) to spray the beauty-care-solution mixture.

5. The beauty care system (40) according to claim 3, **characterized in that** each container portion (411) further includes:
a piston (4113) and a high-pressure gas (4114) disposed in the container (4111),
wherein the high-pressure gas (4114) is located on one side of the piston (4113), and the beauty-care solution is located on the other side of the piston (4113); and
when a pressure of high-pressure gas is greater than a pressure of the beauty-care solution, the high-pressure gas pushes the piston (4113) toward the beauty-care solution to push the beauty-care solution into the collecting portion (412).

6. The beauty care system (40) according to claim 3, **characterized in that** each container portion (411) further includes:
a piston (4113) disposed within the container (4111), and a driving portion (4114),
wherein one end of the driving portion (4114) protrudes into the container (4111) and is connected to the piston (4113) for driving the piston (4113) to move within the container (4111), so as to push the beauty-care solution into the collecting portion (412).

7. The beauty care system (40) according to claim 2, **characterized in that** the characteristic information includes skin properties of the target object, and the beauty care system (40) further comprises:
a skin detection module configured to detect skin properties of the target object, including one or more of:
a humidity detection unit configured to detect a skin moisture of the target object;
temperature detection unit configured to detect a skin temperature of the target object;
a pH detection unit configured to detect a skin pH value of the target object, and
a skin oiliness detection unit configured to detect a skin oiliness of the target object.

8. The beauty care system (40) according to claim 2, **characterized in that** the characteristic information further includes:
time information to perform the beauty care operation on the target object;
geographic information of a location of the target object; and
environment information of the location of the target object, including one or more of temperature and humidity.

9. The beauty care system (40) according to claim 2, **characterized in that** it further comprises one or more of:
a cooling or heating module configured to perform a cooling or hearing operation on the target object,
a vibration module configured to perform a vibration operation on the target object, and
a light irradiation module configured to emit light on the target object; and
wherein the control information further includes one or more of:
second-type information used for controlling the cooling or heating module to perform the cooling or heating operation,
third-type information used for controlling the vibration module to perform the vibration operation, and
fourth-type information used for controlling the light irradiation module to emit light.

10. A method for determining control information for controlling a beauty care system (40), for example for controlling the beauty care system (40) of one of claims 1 to 9 , comprising, by the user terminal:
establishing a communication link with the beauty care system (40);
determining information of an electronic tag of a cosmetic capsule,
**characterized in that** the information includes at least one of information on whether the cosmetic capsule can be used and what kind of beauty care mode is recommended for using the beauty capsule; and **in that** the method further comprises, by the user terminal:
obtaining characteristic information of a target object; and
based on at least one of the information of the electronic tag and the characteristic information of the target object, determining control information for the beauty care system (40) to perform a beauty-care operation on the target object.

11. The method according to claim 10, **characterized in that** the method further comprises by the user terminal:
transmitting the control information to the beauty care system (40) to cause the beauty care system (40) to perform the beauty-care operation on the target object

12. The method according to claim 10 or 11, **characterized in that** the determining information of the electronic tag further includes:
identifying an RFID tag of the cosmetic capsule to read the information from the RFID tag.

13. The method according to one of claims 10 to 12, **characterized in that** the characteristic information includes skin properties of the target object, and the obtaining the characteristic information of the target object further includes:
(i) using a skin detection module (42) to perform a skin detection on the target object to obtain the characteristic information of the target object, and/or
(ii) causing the beauty care system (40) to perform a skin detection using a skin detection module (42) of the beauty care system (40) to detect skin properties of the target object; and receiving the skin properties from the beauty care system (40),
wherein the skin properties include at least one of a skin moisture, a skin temperature, a skin pH value, and a skin oiliness.

14. The method according to one of claims 10 to 13, **characterized in that** it further comprises:
acquiring geographic environment information of the target object,
wherein the geographic environment information includes geographic information of a location of the target object and environment information of the location of the target object, including one or more of temperature and humidity.

15. The method according to one of claims 12 to 14, **characterized in that** determining the control information for the beauty care system (40) further includes:
determining a desired beauty care mode matching the cosmetic capsule according to the information of the electronic tag of the cosmetic capsule; and
based on the desired beauty care mode and the characteristic information of the target object, determining the control information of the beauty care operation of the target object,
wherein the control information include at least one of:
a first-type information used for controlling a beauty-care-solution-mixing control module (301) of the beauty care system (40) to generate a beauty-care-solution mixture to be applied on the target object;
a second-type information used for controlling a cooling or heating module (302) of the beauty care system (40) to perform a cooling or heating operation on the target object;
a third-type information used for controlling a vibration module (303) of the beauty care system (40) to perform a vibration operation on the target object; and
a fourth-type information used for controlling a light irradiation module (44, 304) of the beauty care system (40) to emit light on the target object,
wherein:
the beauty-care-solution-mixing control module (302) includes:
one or more container portions (411) each contain a beauty-care solution;
a collecting portion (412) coupled to the container portions (411) to collect the beauty-care solutions outputted from the container portions (411);
an agitating portion (413) coupled to the collecting portion (412) for agitating the beauty-care solutions in the collecting portion (412) to form a beauty-care-solution mixture; and
a spraying portion (414) for spraying the beauty-care-solution mixture on the target object,
wherein each container portion (411) includes a container (4111) configured to contain the beauty-care solution; and an actuator (404, 4112) fixed to one end of the container (4111) and configured to control the beauty-care solution outputted into the collecting portion (412), and
the first-type information further includes:
a first parameter for controlling each actuator (404, 4112) to the flow of the beauty-care solution in the container (4111) into the collection portion (412) by instructing whether the actuator (404, 4112) is to be activated and a time duration during which the actuator (404, 4112) is activated;
a second parameter for controlling the agitating portion (403) to agitate the beauty-care solutions in the collecting portion (412); and
a third parameter for controlling the spraying portion (414) to spray the beauty-care-solution mixture.
